# EUROPEAN PATENT APPLICATION

(11) **EP 1 961 766 A1**
(43) Date of publication of application: **27.08.2008**
(21) Application number: 06755342.0
(22) Date of filing: 17.05.2006
(51) Int. Cl.: C07K 14/705

(54) **METHOD FOR THE DIAGNOSIS OF ALZHEIMER'S DISEASE**

(30) Priority: 16.06.2005 ES 200501469
(71) Applicant: Fina Biotech, S.L.U., 28223 Pozuelo de Alarcón (Madrid) (ES)
(72) Inventor: FERRER ABIZANDA, Isidro, E-28223 Pozuelo De Alarcón (Madrid) (ES); BARRACHINA CASTILLO, Marta, E-28223 Pozuelo De Alarcón (Madrid) (ES); SUBIRADA SOL , Francisco, E-28223 Pozuelo De Alarcón (Madrid) (ES); DURANY TURK, Olga, María, E-28223 Pozuelo De Alarcón (Madrid) (ES); BUESA ARJOL, Carlos, Manuel, E-28223 Pozuelo De Alarcón (Madrid) (ES); MAES, Tamara, E-28223 Pozuelo De Alarcón (Madrid) (ES)
(74) Representative: Carpintero Lopez, Francisco
(86) International application number: PCT/ES2006/000255
(87) International publication number: WO 2006/134185

(57) **Abstract**

The invention relates to a method for the diagnosis and/or prognosis of Alzheimer's disease, consisting in determining the expression level of a gene encoding a lysosomal marker.

## Description

### FIELD OF THE INVENTION

The field of application of the present invention is within the health sector, mainly that related to neurodegenerative disease, and it is specifically aimed at methods for diagnosing Alzheimer's disease.

### BACKGROUND OF THE INVENTION

Alzheimer's disease (AD) is considered to be the main cause of dementia, the latter being the fourth cause of death in developed countries (1). It is defined as a neurodegenerative suffering of the central nervous system and is characterized by a progressive deterioration of higher brain functions.

AD is microscopically characterized by the presence of senile plaques (diffuse and classic), neurofibrillary tangles, neuropil threads, neuronal degeneration, βA-amyloid protein deposits, granulovacuolar degeneration and the presence of Hirano bodies, among other pathologies (2).

Clinical criteria that have been well established by the fourth edition of the Diagnostic and Statistical Manual of the American Psychiatric Association (DSM-IV) (3) or by the National Institute of Neurologic, Communicative Disorders and Stroke - Alzheimer's Disease and Related Disorders Association (NINCDS-ADRDA) (4) are used to diagnose AD. However, the greatest dilemma of these clinical studies is the diagnostic certainty. Currently, the only way to confirm the clinical diagnosis is to conduct a postmortem analysis in brain tissue to find the existence of neurofibrillary tangles and plaques.

Different genetic markers have recently been studied for their application in the diagnosis of AD such as:
- determination of mutations in the amyloid precursor protein (APP) gene, mutations in the presenilin-1 (PS1) gene and presenilin-2 (PS2) gene, only valid for a reduced number of AD cases with an early or familial occurrence. (5).
- genetic value of the ApoE genotype, which is only determined in those cases complying with the clinical criteria of probable AD, the problem is that is gives a high number of false positives.

Apart from these genetic markers, there are biochemical markers such as:
- Tau protein: this protein is determined by means of neuronal antibodies that can detect tau in cerebrospinal fluid, however, tau levels in AD are not related to age, sex, evolution of the disease, or to the degree of dementia, in addition to detecting high tau levels in other pathologies such as meningitis, meningeal infiltrations, frontal dementias and Creutzfeldt-Jakob.
- βA-amyloid protein: this protein lacks diagnostic usefulness in sporadic forms of AD (6).

There is currently still not any scarcely invasive diagnostic instrument with suitable sensitivity, specificity and predictive value for Alzheimer's disease. This disease further involves an enormous social cost due to, among others, the inability of the patients to take care of themselves, therefore there is a need for a reliable diagnostic method by means of markers which allows preventing the disease, improving the treatment and predicting the evolution of the disease.

### BRIEF DESCRIPTION OF THE INVENTION

The present invention provides a method for the diagnosis and/or prognosis of Alzheimer's disease by means of determining the expression level of a gene encoding a lysosomal marker. The lysosomal marker is preferably Lamp-1 or Lamp-2.

A particular aspect of the invention consists of an *in vitro* method for the diagnosis and/or prognosis of Alzheimer's disease by means of determining the expression level of a gene encoding a lysosomal marker in a biological sample and comparing said level with a reference value, in which the alteration of said level is indicative of Alzheimer's disease and of the stage of said disease.

"Reference value" in the present invention designates levels of mRNA and of the protein encoded by the lysosomal marker gene present in a healthy individual who does not suffer from AD or other diseases affecting levels of the mRNA or of the protein encoded by said lysosomal marker gene.

According to a preferred embodiment of the present invention, the gene encoding the lysosomal marker is preferably Lamp-1 or Lamp-2.

According to a preferred embodiment of the present invention, the biological sample comprises a tissue, said tissue is preferably a tissue homogenate. The tissue homogenate is preferably obtained from nervous tissue cells or peripheral neuroendocrine cells.

According to another preferred embodiment of the invention, the biological sample is a biological fluid, said biological fluid preferably comprises cerebrospinal fluid, blood or serum.

The determination of the expression level of the gene encoding a lysosomal marker is carried out in a particular embodiment by means of measuring the amount of mRNA encoded by said gene or fragments thereof. The lysosomal marker gene is preferably Lamp-1 or Lamp-2.

The analysis of the amount of mRNA encoded by said gene or fragments thereof is preferably carried out by means of amplification, using oligonucleotides specific for PCR, SDA or any other cDNA amplification method allowing a quantitative estimation of the levels of the lysosomal marker transcript.

The analysis of the amount of mRNA encoded by said gene or fragments thereof is preferably carried out by means of DNA biochips made with oligonucleotides deposited by any mechanism known by a person skilled in the art or synthesized *in situ* by means of photolithography or by means of any other mechanism known by a person skilled in the art.

According to another preferred embodiment of the invention, the determination of the expression level of the gene encoding a lysosomal marker is carried out means of measuring the amount of protein encoded by said gene or of fragments thereof. The measured protein is preferably Lamp-1 or Lamp-2.

The measurement of the amount of protein encoded by said gene or of fragments thereof is preferably carried out by means of Western-blot.

The measurement of the amount of protein encoded by said gene or of fragments thereof is also carried out by means of protein chips using antibodies or fragments of specific antibodies against the lysosomal marker or by means of protein profiles carried out by mass spectrometry or by any other mechanism allowing a quantitative estimation of the levels of protein of the lysosomal marker.

In another preferred embodiment, the measurement of the amount of protein encoded by said gene or of fragments thereof is carried out by means of immunohistochemical techniques.

Another preferred embodiment of the invention comprises the determination of the expression level of the gene encoding a lysosomal marker by means of image analysis.

According to a more specific embodiment of the invention, the determination of the expression level of the gene encoding a lysosomal marker is carried out by means of image analysis from the quantification on immunohistochemical images. Examples of quantification methods include but are not limited to morphometry, densitometry and fluorescence intensity.

Another aspect of the invention consists of a kit for the diagnosis and/or prognosis of Alzheimer's disease comprising the necessary reagents for carrying out the determination of the expression level of the gene encoding a lysosomal marker, preferably for determining the expression level Lamp-1 or Lamp-2. The kit allows carrying out the method according to the invention which has just been described.

The kit for the diagnosis and/or prognosis of Alzheimer's disease preferably comprises the necessary reagents for the determination of the level of mRNA encoded by the lysosomal marker gene and more preferably comprises the necessary reagents for the determination of the level of mRNA encoded by the lysosomal marker gene by means of amplification.

The kit for the diagnosis and/or prognosis of Alzheimer's disease preferably comprises the necessary reagents for the determination of the level of mRNA encoded by the lysosomal marker gene. It preferably comprises the necessary reagents for the determination of the level of mRNA encoded by the lysosomal marker gene by means of DNA biochips.

On the other hand, the kit for the diagnosis and/or prognosis of Alzheimer's disease can comprise the necessary reagents for the determination of the level of protein encoded by the lysosomal marker gene. It preferably comprises the necessary reagents for the determination of the level of protein encoded by the lysosomal marker gene by means of Western-blot.

The kit for the diagnosis and/or prognosis of Alzheimer's disease preferably comprises the necessary reagents for the determination of the level of protein encoded by the lysosomal marker gene. It preferably comprises the necessary reagents for the determination of the level of protein encoded by the lysosomal marker gene by means of protein chips.

The kit for the diagnosis and/or prognosis of Alzheimer's disease preferably comprises the necessary reagents for the determination of the level of protein encoded by the lysosomal marker gene. It preferably comprises the necessary reagents for the determination of the level of protein encoded by the lysosomal marker gene by means of immunohistochemical techniques.

According to a preferred embodiment of the invention, the determination of the expression level of a gene encoding a lysosomal marker is carried out by means of an reporting substance binding specifically to the mRNA or to the protein encoded by said gene.

"Reporting substance" in the present invention relates to an antibody, a monoclonal antibody, an oligonucleotide, a macromolecule, an organic molecule or generally, any substance which can bind specifically to the mRNA or to the protein encoded by the lysosomal marker gene. Said indicating substance comprises a labeling which can be an enzyme, a radioisotope, a dye, a fluorescent compound, a chemiluminescent compound, a bioluminescent compound, a metal chelate or generally any labeling known in the state of the art which can be detected by means of a detection method.

A particular aspect of the invention is formed by kit for the diagnosis and prognosis of Alzheimer's disease comprising the necessary reagents for carrying out the determination of the expression level of the gene encoding a lysosomal marker comprising a composition containing an reporter substance binding specifically to the mRNA or to the protein encoded by said gene, in which said indicating substance is labeled with a detectable marker and a physiologically acceptable fluid.

A particular aspect of the invention is represented by the use of at least one gene encoding a lysosomal marker selected from Lamp-1 and Lamp-2, as a genetic marker for the diagnosis and prognosis of Alzheimer's disease.

Other aspects of the invention will become evident for persons skilled in the art.

### DESCRIPTION OF THE DRAWINGS

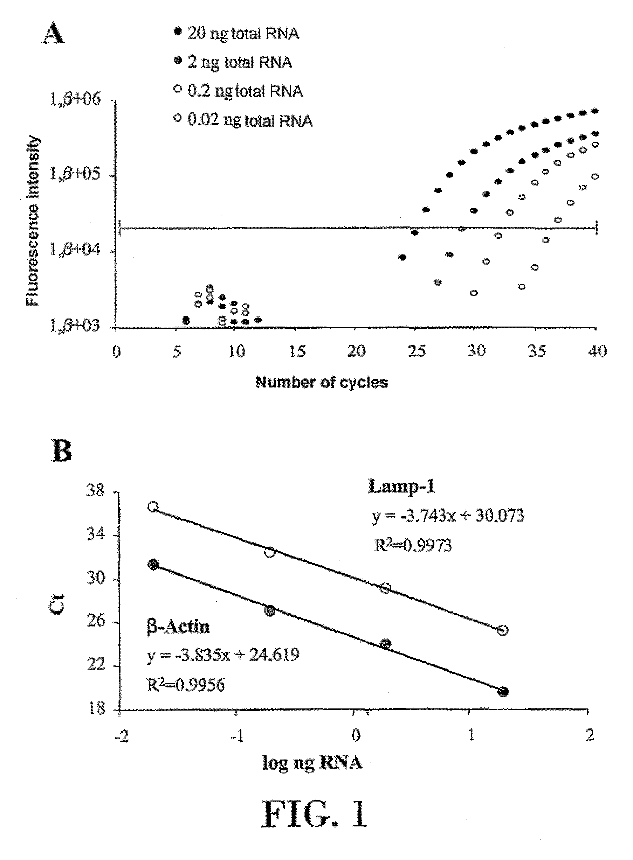

Figure 1A shows the amplification of Lamp-1 using serial dilutions of RNA of control human brains. The horizontal line shows the manually adjusted threshold line in the exponential phase. The fluorescence intensity increases with the increase of PCR cycles. The number of PCR cycles in which the fluorescence intensity exceeded the threshold line was defined as the CT value on which the relative quantification was based.
Figure 1B shows the representative standard curves for β-actin and Lamp-1 constructed from several RNA concentrations of control human brains. The CT values (Y axis) against the logarithm of the RNA concentration of the control samples (X axis) showed an inverse linear correlation.

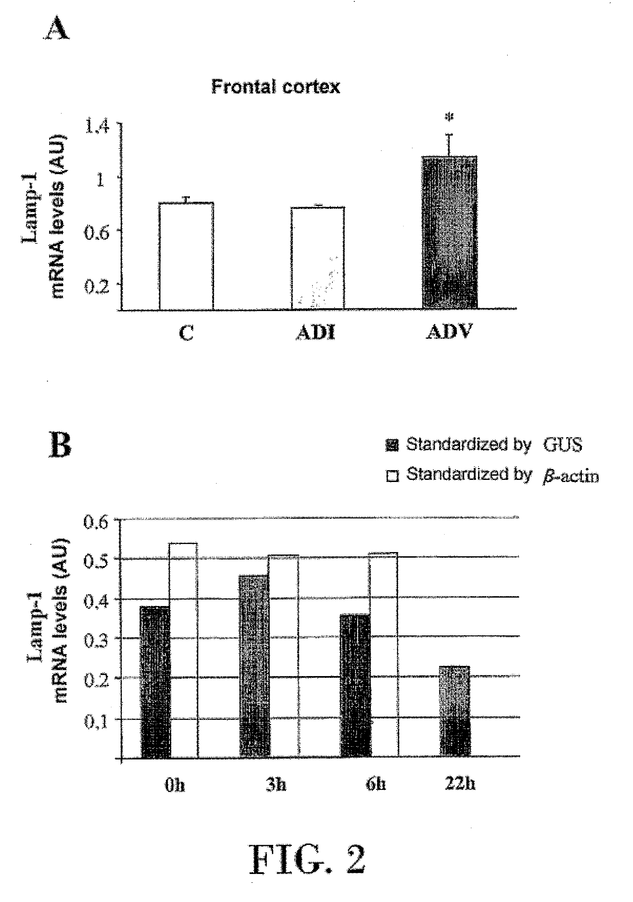

Figure 2A shows the Lamp-1 mRNA levels (mean ± SEM) in the frontal cortex of the control samples (C, n = 4), six AD cases, stages I-IIAIB (according to Braak and Braak) (ADI, n = 6), and five AD cases, stages V-VIC (ADV, n = 5). The Lamp-1 mRNA levels were standardized with β-actin.

Figure 2B shows the Lamp-1 mRNA levels normalized with β-actin and GUS in the frontal cortex of a single sample (3 h post-mortem). The samples were frozen directly in liquid nitrogen (0 h) or left at room temperature for 3, 6 or 22 hours and then frozen in liquid nitrogen.

There is no apparent mRNA degradation until 22 h. *p<0.05 compared with the control samples (ANOVA con post-hoc LSD test).

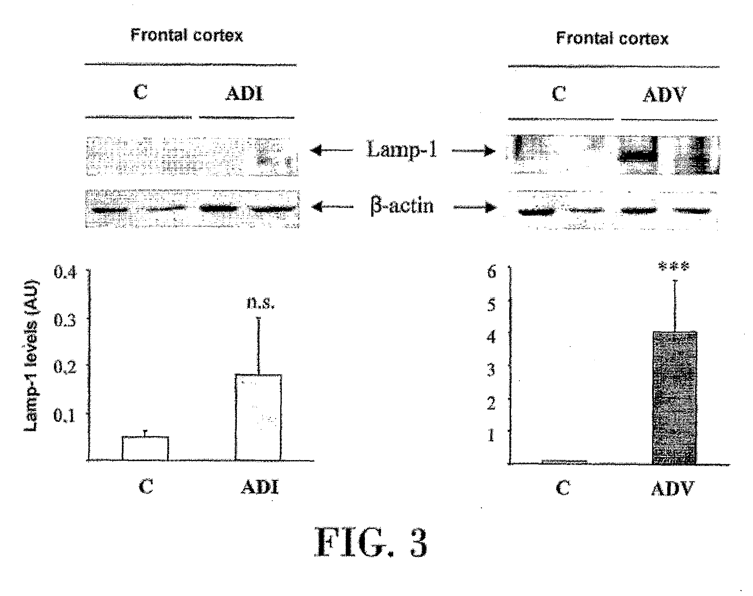

Figure 3 shows Lamp-1 (approximately 125 KDa) as detected by means of Western Blot in frontal cortex homogenates (area 8). β-actin is used as protein charge control. The image is representative of all the samples indicated in Table I. The densitometric analyses of the Lamp-1 protein levels (mean ± SEM) were carried out with the TotalLab v2.01 software. The Lamp-1 protein values were normalized with β-actin. ***p<0.001 compared con the control samples (ANOVA with post-hoc LSD test); n.s.: non-significant differences when it was compared with the controls.

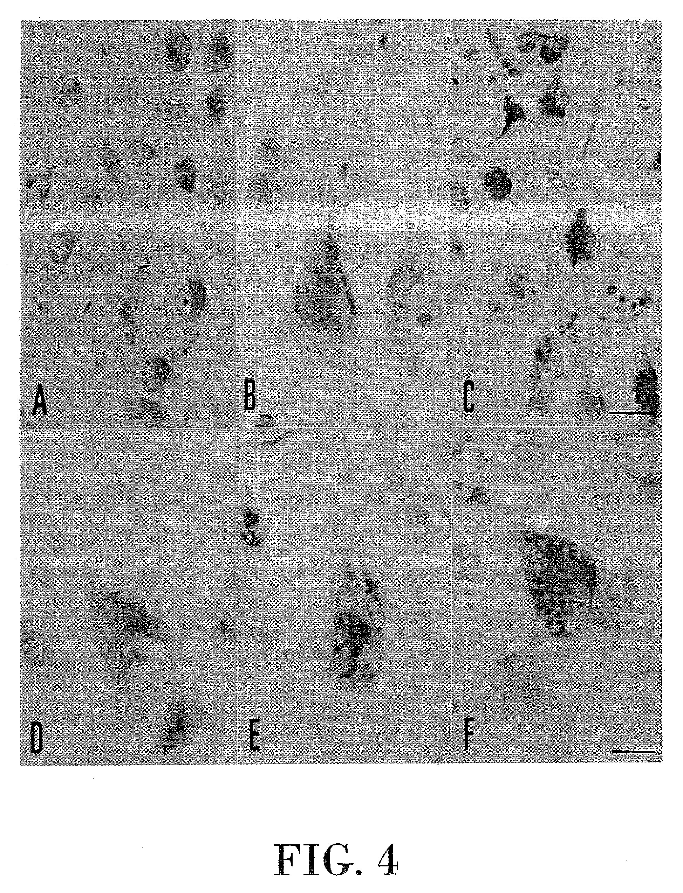

Figure 4 shows the image analysis of the Lamp-1 immunoreactivity in the frontal cortex (A-E) and in the hippocampus (F), in the control (A, B) and in AD cases (stages VC) (C-F). A moderate Lamp-1 immunoreactivity was found in neuron cytoplasm in AD. Neurofibrillary tangles were not stained with Lamp-1 (D, E). Neurons with granulovacuolar degeneration showed a strong Lamp-1 immunoreactivity. A and C, line in C = 50 µm. B, D-F, line in F = 25 µm.

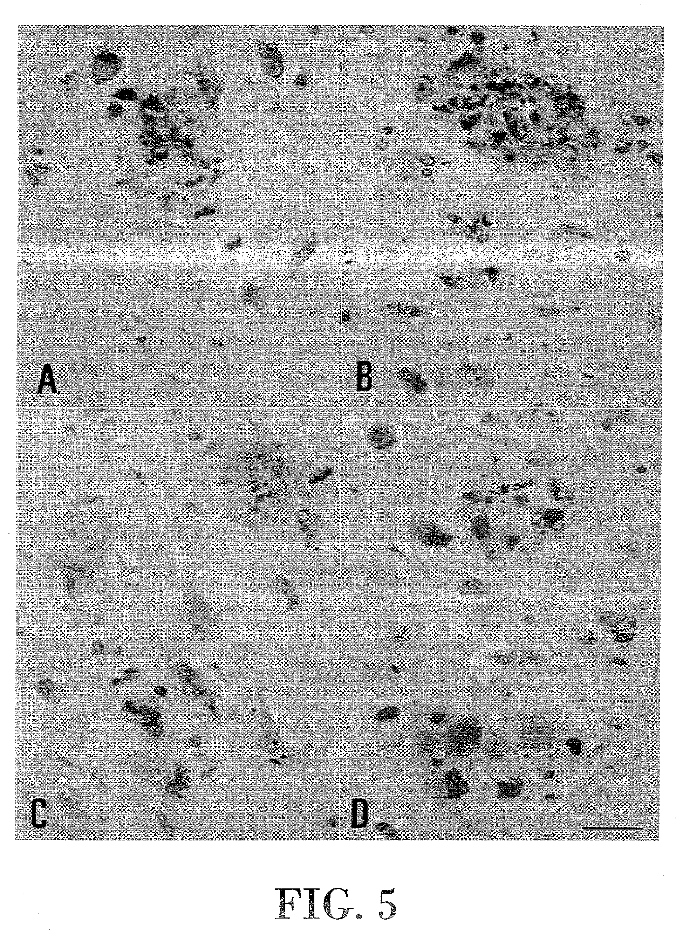

Figure 5 shows the image analysis of Lamp-1 immunoreactivity, indicating that it was located in the cellular process surrounding the amyloid deposits in senile plaques (A-D), in addition to in neurons Line = 50 µm

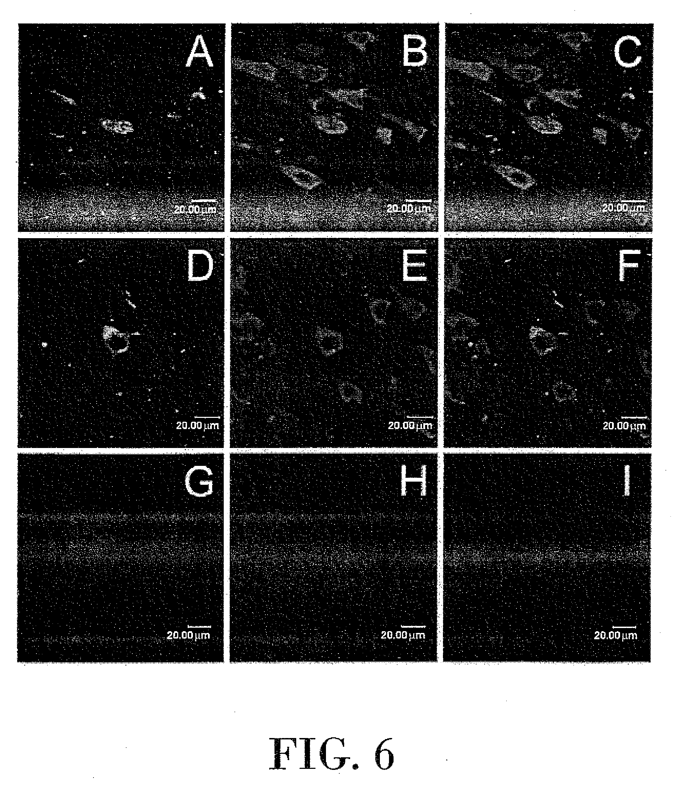

Figure 6 shows the image analysis of the double labeling immunofluorescence for Lamp-1 (green) and for phosphorylated-Tau (AT8 antibody, red) in AD (superimposition C and F, yellow). Most of the neurons with phosphorylated-Tau protein deposits show a low Lamp-1 expression, whereas only a few neurons with strong Lamp-1 immunoreactivity show a phosphorylated-Tau deposit (A, B and D, E). The control sections stained with only secondary antibodies were negative (G-I).

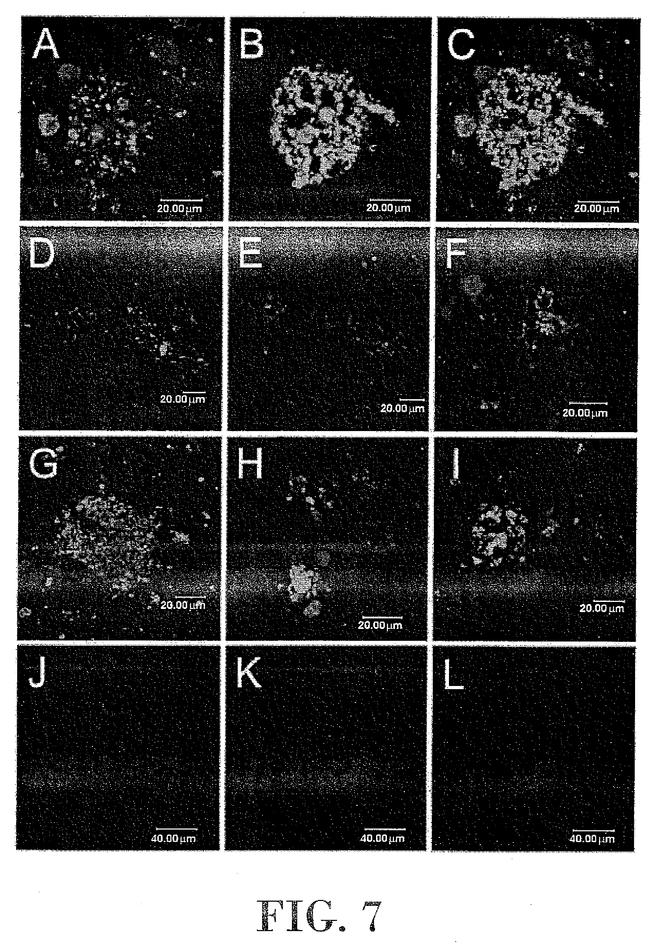

Figure 7 shows the image analysis of the double labeling immunofluorescence for Lamp-1 (green) and for βA-amyloid (red), and confocal microscopy (superimposition, yellow). Immunoreactive Lamp-1 deposits were found around the amyloid plaques (A-C). Lamp-1 immunoreactivity appears with βA-amyloid condensation in the senile plaques. Little or no immunoreactive Lamp-1 profile appeared in the diffuse plaques (D-F), whereas the immunoreactive Lamp-1 process increases in the plaques with amyloid nuclei (G-I). The control sections stained with only secondary antibodies were negative (J-L).

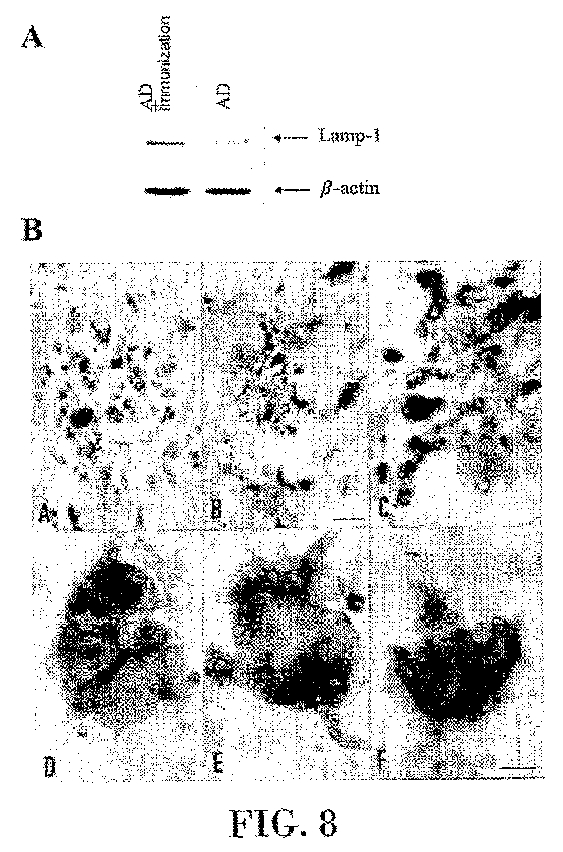

Figure 8A shows Lamp-1 protein levels, analyzed by Western blot, in the cerebral cortex in the case of AD with encephalitis after immunization with βA peptide. No differences were observed in the expression levels when they were compared with AD stage V-VI/C.

Figure B shows the image analysis of Lamp-1 immunoreactivity, indicating that it mainly appeared in microglial cells surrounding the collapsed amyloid deposits (A-C) and in multinucleated giant cells (D-F), the result being the phagocytosis of the βA-amyloid residues. A and B, line in B = 50 µm, C-F, line in F = 25 µm.

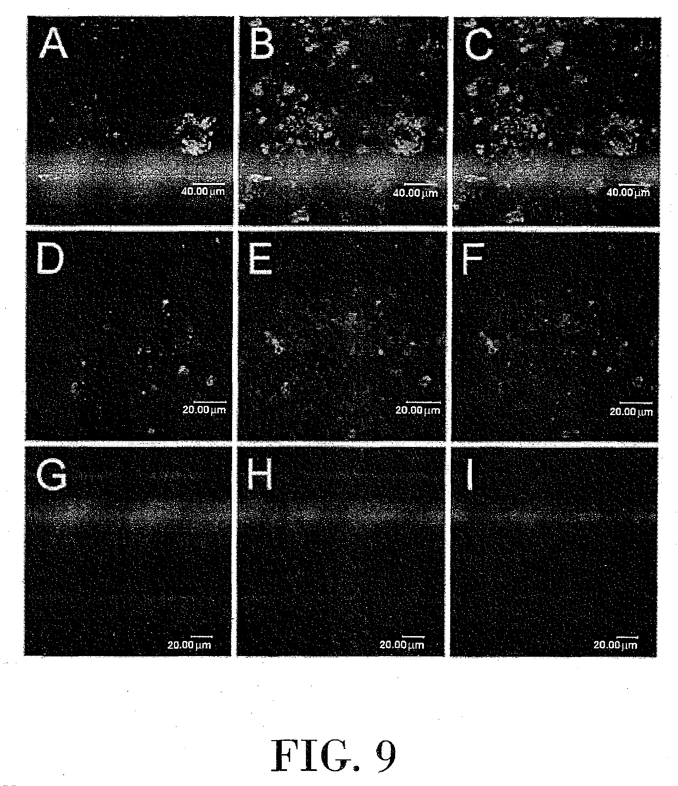

Figure 9 shows the image analysis for the double labeling immunofluorescence for Lamp-1 (green) and for CD68 (red), and confocal microscopy (superimposition, yellow) in the case of AD with encephalitis after immunization with βA peptide. Lamp-1 immunoreactivity was found in microglial cells and multinucleated giant cells (A-F). The control sections stained with only secondary antibodies were negative (G-1).

### DETAILED DESCRIPTION OF THE INVENTION

The present invention provides a method for the diagnosis and prognosis of Alzheimer's disease in a simple and reliable manner, with a great importance for an early diagnosis of the disease and to assess the evolution of the patient with AD.

An experiment was carried out in which the expression levels of Lamp-1 mRNA and of the protein in brain samples of patients with AD and control brain samples were compared.

The brain samples were obtained by the autopsy of 11 patients with AD and 4 controls. The informed consent of the patients or of their relatives was required and the study was approved by ethics committees.

The time between death and tissue processing was 2-10 hours. Half of the brain was cut in 1 cm thick coronal sections and were frozen in dry ice at -80°C until their use.

For the morphological examinations, the brains were fixed by immersion in a 10% formalin buffer for two or three weeks.

The neurological study was carried out in 4 µm thick wax-free paraffin sections of the frontal (area 8), primary motor, primary sensor, parietal, superior temporal, inferior temporal, anterior cingulate, anterior insular and visual associative and primary cortex; entorhinal cortex and hippocampus; caudate" putamen and pallidum; mid and posterior thalamus; subthalamus; Meynert's nucleus; amygdala; mesencephalon, pons and bulb; and cerebellar cortex and dentate nucleus.

The sections were stained with hematoxylin and eosin, luxol fast blue with the Klüver Barrera method and for the immunohistochemistry of the acid proteins of glial fibers, CD 68 and tomato lecithin for microglia, (βA-amyloid, pan-tau, tau specifically phosphorylated at Thr181, Ser202, Ser214, Ser262, Ser396 and Ser422 and αB-crystalline, α-synuclein and ubiquitin.

The AD stages were established according to the amyloid deposit charge and according to the neurofibrillary pathology following the Braak and Braak classification (7):
Stage A: initial deposits in the basal neocortex
Stage B: deposits extended in neocortex-associated areas
Stage C: large deposits in the entire cortex
I-II: neurofibrillary pathology stages in transentorhinal
III-IV: limbic
V-VI: neocortical

6 patients were classified as AD I-IIA/B, and 5 as AD V-VIC. The main clinical and neuropathological data are summarized in Table 1. These patients did not present any associated pathology (vascular, synucleinopathy). The brain of an AD case presenting encephalitis after the immunization with amyloid peptide (11) was included in the study for comparative purposes.

Frontal cortex samples of a control individual were further included, these samples were obtained 3 hours postmortem, some were immediately frozen and others were stored at 4°C for 3 hours, 6 and 22 hours and they were later frozen to limit the variable postmortem delay in the processed tissues and its effect in preserving Lamp-1 mRNA

Once the samples have been prepared, the determination of both Lamp-1 mRNA and of the protein was carried out and biochemical, immunohistochemical and microscopic studies were conducted as described in the examples. The control and diseased brain samples were processed in parallel. The results of these studies show that there is an increase of the expression levels of Lamp-1 mRNA and of the protein in the cerebral cortex in advanced AD stages. Lamp-1 is further located in the cytoplasm of neurons and in dystrophic neuritis surrounding senile plaques.

### EXAMPLES

The following examples are useful for illustrating but not limiting the present invention.

### Example 1: mRNA isolation and confirmation of the results by cDNA synthesis and TaqMan PCR

Total RNA was isolated using Trizol Reagent® (Life Technologies) followed by RNeasy Protect Mini Kit (Qiagen). The frozen human brain tissues were directly homogenized in 1 ml of Trizol per 100 mg of tissue. The total RNA was extracted using the protocol suggested by the supplier. The purified total RNA was then resuspended in 100 µl of RNase-free water, the mRNA was purified following the RNeasy Protect Mini Kit protocol with minimal modifications. Treatment with DNase was discarded due to the elimination of genomic DNA during the extraction with Trizol. The concentration of each sample was measured at A₂₆₀, and the RNA integrity was confirmed by means of formaldehyde-agarose gel electrophoresis.

cDNA synthesis and TaqMan PCR were then carried out. For each 100 µl of reverse transcription reaction, 2 µg of human RNA with 2.5 µM random hexamers, RT TaqMan 1x buffer, 5.5mM MgCl₂, 500 µM of each dATP, dTTP, dCTP and dGTP, 0.4 U/µl of RNase inhibitor and 1.25 U/µl MultiScribe reverse transcriptase (Applied Biosystems). The reactions were carried out at 25°C for 10 minutes to maximize the binding of mold RNA to the primer, followed by 30 min. at 48°C and then by incubation for 5 min. at 95°C to deactivate the reverse transcriptase. To check the degree of contamination by genomic DNA, parallel reactions were carried out for each RNA sample in the absence of MultiScribe reverse transcriptase.

The TaqMan probe (Applied Biosystems) binds to the mold DNA strand between the direct and reverse primers. The probe contains an reporter stain which is released by the Taq polymerase during amplification, therefore the fluorescence generated will be proportional to the amount of product accumulated.

β-actin and β-glucuronidase (GUS) were used as internal controls. The primers and the specific fluorescent probes for Lamp-1, GUS and β-actin were purchased from Taq-Man Gene Expression Assays (Applied Biosystems).

The TaqMan PCR assays for the Lamp-1 and for the internal controls were carried out in triplicate on cDNA samples in 96-well plates on an ABI Prism 7700 Sequence Detection system (PE Applied Biosystem). For each 20 µl of TaqMan reaction, 9 µl of cDNA (diluted 1/50, corresponding to approximately 4 ng of RNA) was mixed with 1 µl of 20x TaqMan Gene Expression Assays and 10 µl of 2x TaqMan Universal PCR 30Master Mix (Applied Biosystem). Parallel studies were carried out for each sample using primers and probes with β-actin and GUS for the standardization. The reaction was carried out using the following parameters: 50°C for 2 minutes, 95°C for 10 minutes and 40 cycles at 95°C for 15 seconds and 60°C for 1 minute. The standard curves were prepared for Lamp-1 and for each internal control using dilutions in series of human RNA control samples. The TaqMan PCR data were finally captured using Sequence Detector Software (SDS version 1.9; Applied Biosystem).

The increase of Lamp-1 mRNA levels were confirmed by means of TaqMan PCR assays with the control brain samples and with AD. The ABI 7700 measures the fluorescence accumulation of the PCR products by the continuous monitoring of the cycle threshold (Ct) which is an arbitrary value that is manually assigned to a level above the baseline, in the exponential phase of the PCR in which there is no limiting factor. The Ct value establishes the point at which the sample amplification crosses the threshold (Figure 1A). For each sample the amount of target and of internal controls is determined from the standard curves which were plotted showing Ct (Y) versus the log of the ng of total control RNA. The amount of each Lamp-1 target was divided by the amount of the internal controls to obtain a standardized target value which allowed determining the relative Lamp-1 mRNA levels in the control samples and in the pathological samples. The levels of the internal controls used to standardize Lamp-1 MRNA values were not modified in the pathological samples compared to the controls and were further similar between the different pathologies.

The results showed a relative increase of the Lamp-1 mRNA levels in the frontal cortex in AD belonging to stages V-VIC when compared with the controls: P<0.05, ANOVA with the LSD test (post-hoc Fisher's least significant difference) (Figure 2A). The Lamp-1 mRNA levels in the frontal cortex were not modified in the early stages of the AD (Stages I-IIA/B) (Figure 2A) in comparison to the control values.

### Example 2: Electrophoresis gel and Western blotting

The frozen frontal cortex samples (area 8, 100 mg) were directly homogenized in 1 ml of lysis buffer (20 mM Hepes, 10 mM KCI, 1.5 mM MgCl2, 1 mM EDTA, 1 mM EGTA, 1 mN DDT, 2 mM PMSF, 1 µg/ml aprotinin, leupeptin and pepstatin) and subjected to sonication. The lysates were centrifuged at 5,000 rpm for 10 minutes at 4°C and the protein concentration was determined by means of the BCA method (Pierce). 50 µg of total proteins were subjected to 95°C and were then loaded into SDS-polyacrylamide gels with a Tris-glicine elution buffer. The proteins were subjected to electrophoresis using a Mini-Protein system (Bio-Rad) and transferred to nitrocellulose membranes (Bio-Rad) with a Mini Trans Blot electrophoresis transfer cell (Bio-Rad) for 1 hour at 100 V. The nitrocellulose membranes were blocked with Tween 20 TBS (TBST) containing 5% skimmed milk for 30 minutes. The membranes were then incubated overnight at 4°C with one of the primary antibodies in TBST with 3% BSA. The following antibodies were used: anti-Lamp-1 antibody (H-228, sc-5570, Santa Cruz) 1:1000 dilution, anti-β-actin antibody (AC-74 clone, Sigma) 1:5000 dilution. After the incubation with the primary antibody, the membranes were washed three times with TBST for 5 minutes at room temperature and were then incubated with rabbit and mouse anti-IgG antibodies labeled with radish peroxidase (Dako), 1:1000 dilution (1:5000 for β-actin) for one hour at room temperature. The membranes were then washed 4 times, 5 minutes each time with TBST at room temperature and developed with the ECL Western blotting chemiluminescence system (Amersham/Pharmacia), the membranes were then exposed to autoradiographic film (Hyperfilm ECL, Amersham).

The densitometric quantification of the Western Blot bands was carried out by means of the TotalLab v2.01 software. The Statgraphics Plus v5 was used for the statistical analysis.

The results showed that the Lamp-1 protein levels, like the Lamp-1 mRNA levels, were increased in the frontal cortex in the AD stages V-VIC (p<0.001, ANOVA with LSD test), but were not increases in the early AD stages I-IIA/B in comparison to the control samples (Figure 3).

### Example 3: Immunohistochemistry

The 5µm thick wax-free sections of the frontal cortex, hippocampus and entorhinal cortex were processed for immunohistochemistry according to the labeled streptavidin-biotin peroxidase (LSAB) method. After the incubation with methanol and H₂O₂ in PBS and normal serum, the sections were incubated with anti-Lamp-1 antibody (Santa Cruz) in a 1:100 dilution. After the incubation with the primary antibody, the sections were incubated with LASB for 15 minutes at room temperature. The peroxidase reaction was viewed with diaminobenzidine and H₂O₂. The immunostaining control included the omission of the primary antibody, no signal was obtained by following the incubation with the secondary antibody exclusively. The sections were slightly stained with hematoxylin.

The moderate Lamp-1 immunoreactivity characterized by small cytoplasmic granules was found in neurons and microglial cells in control samples (Figure 4A, B), whereas an increase of Lamp-1 immunoreactivity was found in cortical neurons in AD (Figure 4C).

Curiously, this increase was not associated to the NFT pathology individual neurons, since Lamp-1 immunoreactivity was not found in tangles (Figure 4D, E). An increase of Lamp-1 immunoreactivity was also found in neurons with granulovacuolar degeneration in the hippocampus (Figure 4F), and a strong Lamp-1 immunoreactivity was found in neuritic plaque cells (Figure 5), being limited in the frontal cortex of the AD stages I-IIB (case 8), but very visible in the frontal cortex of AD stages V/VIC.

### Example 4: Double labeling immunofluorescence and confocal microscopy

The wax-free sections of the frontal cortex were stained with a saturated Sudan black B solution (Merck) for 30 minutes to block the autofluorescence of the lipofuscin granules present in the neuron bodies, rinsed in 70% ethanol and washed with distilled water. The sections were incubated overnight at 4°C with a mouse polyclonal anti-Lamp-1 antibody (Santa Cruz) using a 1:100 dilution and the monoclonal βA-amyloid antibody (Dako) 1:50 dilution, mouse antiAT8 antibody (Innogenetics, Gent) 1:50 dilution, or CD68 (Dako) 1:100 dilution. The sections were then washed in PBS, and incubated in the dark with the secondary antibody cocktail and diluted in the same carrier solution as the a primary antibodies for 45 minutes at room temperature. The secondary antibodies were: rabbit Alexa488 (green) and mouse Alexa 546 (red) (both from Molecular Probes, OR) in a 1:400 dilution. After washing with PBS, the sections were mounted in Immuno Fluore mounting medium (ICN Biomedicals, Barcelona), sealed and dried overnight. The sections were examined with the Leica TCS-SL confocal microscope.

The absence of co-localization of Lamp-1 expression and neurofibrillary degeneration, as shown with the AT8 antibody, was also shown with double labeling immunofluorescence and confocal microscopy. The neurons showing strong Lamp-1 immunoreactivity had low immunoreactive phosphorylated-tau protein deposits, whereas the neurofibrillary plaque neurons showed a low Lamp-1 immunoreactivity (Figure 6).

The strong immunoreactivity associated to the plaques was restricted to the cellular process surrounding the central areas of the amyloid, as was disclosed in the immunostained sections with Lamp-1 and βA-amyloid. Lamp-1 immunoreactivity was rarely observed in association with the diffuse plaque, although some immunoreactivity occurred in βA-amyloid condensation processes (Figure 7).

### Lamp-1 immunoreactivity in AD with encephalitis after immunization with βA

AD patients with encephalitis after immunization with βA showed a reduced cerebral amyloid load compared with conventional AD patients, a reduced or absent phosphorylated-tau in the remaining plaques and a marked activation of the microglia and in multinucleated giant cells with βA-amyloid residues (13,19). The expression levels in Western blot were similar to conventional AD in this case (Figure 8A).

However, in accordance with neuropathological observations, Lamp-1 immunoreactivity was found in microglial cells surrounding dense βA-amyloid plaques and in multinucleated giant cells (Figure 8B)

The Lamp-1 expression tests were checked by double labeling immunofluorescence, Lamp-1 and CD68, used as a marker for microglia and multinucleated giant cells, examining them with a confocal microscope (Figure 9). DISCUSSION

The data show an increase of Lamp-1 in the cerebral cortex in AD cases, the mRNA and protein levels of which increase with the progression of said disease. Immunohistochemical techniques, double labeling immunofluorescence and the confocal microscope showed a Lamp-1 localization predominantly in neuritis surrounding the amyloid plaques. The lysosomal hydrolases were located in perikaryon and proximal dendrites in cortical neurons of AD brains, and high levels in senile plaques (8, 9).

There are several studies suggesting that lysosomal disturbances promote the βA-amyloid deposit in AD brains (10, 15) and that the mutations in presenilin are associated to an accelerated neuronal lysosomal pathology (17). Lamp-1 expression is more related to the dystrophic neuritis of senile plaques then with diffuse amyloid deposits, although the Lamp-1 immunoreactivity process also occurs in diffuse cortical plaques, in the cerebellum and in diffuse striatum plaques (9). In this sense, it must be noted that dendrites and synapses in AD contain an increased number of lysosomes (12).

Cathepsin D has also been involved in Tau protein degradation, suggesting a role of the lysosomes in the degradation of neurofibrillary tangles in AD (13). In relation to this, transgenic mice with a triple mutation in the Tau protein show an increase in the number of lysosomes showing an aberrant morphology, as well as a hyperphosphorylated Tau deposit in cortical neurons and in the hippocampus (14). These data suggest that lysosomal abnormalities can be the reason for the degeneration of neurons with hyperphosphorylated Tau deposits.

The results show an inverse relationship between Lamp-1 expression and hyperphosphorylated Tau deposit in cortical neurons with neurofibrillary tangles in AD, although a marked immunoreactivity was found in the neurons with granulovacuolar degeneration.

The study of the case of AD with encephalitis after immunization with βA shows revealing data. Neuropathological studies in a limited number of cases show a reduced amyloid load and reduced amyloid plaques together with an activation of the microglia and with the presence of multinucleated giant cells full of amyloid residues; hyperphosphorylated Tau protein was not observed in collapsed plaques, although the neurons with neurofibrillary tangles are not affected (11, 16). Lamp-1 immunoreactivity was not found in the neuronal process after immunization with βA, but it was found in activated microglial cells and in multinucleated giant cells containing amyloid and cell residues.

**Table I: Main clinical and pathological data of the study. AD stages according to Braak and Braak classification and controls; M: Male, F: Female; NFT: neurofibrillary tangles**

| Patient | Disease | Gender | Age (years) | Disease duration (years) | Post-mortem (hours) | Braak stages | |
|---|---|---|---|---|---|---|---|
| | | | | | | βA4-amyloid | NFT |
| 1 | Control | F | 73 | - | 5 | | |
| 2 | Control | M | 75 | - | 6 | | |
| 3 | Control | F | 79 | - | 7 | | |
| 4 | Control | F | 80 | - | 3 | | |
| 5 | AD | M | 59 | - | 7 | A | I-II |
| 6 | AD | M | 69 | - | 10 | A | I-II |
| 7 | AD | F | 73 | - | 4 | A | I-II |
| 8 | AD | M | 78 | - | 7 | B | I-II |
| 9 | AD | F | 84 | - | 5 | A | I-II |
| 10 | AD | F | 88 | - | 5 | A | I-II |
| 11 | AD | M | 69 | 8 | 6 | C | V |
| 12 | AD | F | 82 | 13 | 10 | C | V |
| 13 | AD | F | 84 | 10 | 2 | C | VI |
| 14 | AD | F | 86 | 8 | 10 | C | VI |
| 15 | AD | M | 93 | 11 | 7 | C | V |

### REFERENCES

1: Pappolla MA. La Neuropatologia y la Biologia Molecular de la Enfermedad de Alzheimer. pp. 543-553. In: Neuropatologia. Diagnóstico y Clínica. Cruz-Sánchez FF. Ed. Edimsa. 2000.
2: Cruz-Sánchez FF et al. Neuropathological Diagnostic Criteria for Brain banking. Ed. IOS Press. 1995.
3: American Psychiatric Association. Diagnostic criteria from DSM-IV. Washington DC:APA; 1994
4: Mc Khann G, Drachman D, Folstein M, Katzman R, Price D, Stadlan EM. "Clinical diagnosis of Alzheimer's Disease: report of the NINCDS ADRDA work group under the auspices of Department of Health and Human Services Task Force on Alzheimer's Disease. Neurology 1984;34:939-44.
5: Gil Nécija Eulogio. Diagnóstico biológico. Cuarto curso nacional de Enfermedad de Alzheimer. Sevilla, 23-24 September 1999. Ed. Andrómico.
6: Guierá A. et al. "Actualización sobre la patología de la enfermedad de Alzheimer". Rev Esp Pato] 2002; Vol 35, no. 1:21-48.
7: H. Braak and E. Braak, Temporal sequence of Alzheimer's disease related pathology. In: A. Peters and J.H. Morrison, (eds.), "Cerebral cortex, vol. 14, Neurodegenerative and age-related change in structure and function of cerebral cortex", Kluwer Academic/Plenum Publishers, New York, Boston, Dordrecht, London, Moscow, 1999, 475-512
8: A.M Cataldo, P.A. Paskevich, E. Kominami arid R.A. Nixon. "Lysosomal hydrolases of different classes are abnormally distributed in brains of patients with Alzheimer disease". Proc. Natl. Acad. Sci. USA 88 (1991) 10998-11002.
9: A. M. Cataldo, J.L. Barnett, D.M. Mann and R.A. Nixon. "Co-localization of lysosomal hydrolase and beta-amyloid in diffuse plaques of the cerebellum and striatum in Alzheimer's disease and Down's syndrome". J. Neuropathol. Exp. Neurol. 55 (1996) 704-715.
10: A. M. Cataldo, J.L. Barnett, C. Pieroni and R.A. Nixon. "Increased neuronal endocytosis and protease delivery to early endosomes in sporadic Alzheimer's disease: neuropathologic evidence for a mechanism of increased beta-amyloidogenesis". J. Neurosci. 17 (1997) 6142-6151.
11 I. Ferrer, M. Boada, M.L. Sánchez, M.J. Rey and F. Costa-Jussa. "Neuropathology and pathogenesis of encephalitis following amyloid-13 immunization in Alzheimer's disease". Brain Pathol. 14 (2004) 11-20.
12: N.K. Gonatas, W. Anderson and I. Evangelista I. " The contribution of altered synapses in the senile plaque: An electron microscopic study in Alzheimer's dementia". J. Neuropathol. Exp. Neurol. 26 (1967) 25-39.
13: A. Kenessey, P Nacharaju, L.W. Ko and S.H. Yen. "Degradation of tau by lysosomal enzyme cathepsin D: implication for Alzheimer neurofibrillary degeneration". J. Neurochem. 69 (1997) 2026-2038.
14: F. Lim, F. Hernandez, J.J. Lucas, P. Gómez-Ramos, M.A. Moran and J. Avila. "FTDP-17 mutations in tau transgenic mice provoke lysosomal abnormalities and tau filaments in forebrain". Mol. Cell Neurosci. 18. (2001) 702-714.
15: P.M. Mathews, C.B. Guerra, Y.Jiang, O.M. Grbovic, B. H. Kao, S.D. Schmidt, R. Dinakar, M. Merchken, A. Hille-Rehfeld, J. Rohrer, P. Mehta, A.M. Cataldo and R.A. Nixon. "Alzheimer's disease-related over-expression of the cation-dependent mannose 6-phosphate receptor increases Aβ secretion: role for altered lysosomal hydrolase distribution in β-amyloidogenesis". J. Biol. Chem. 277 (2002) 5299-52307.
16: J.A.R. Nicoll, D. Wilkinson, C. Holmes, P. Steart, H. Markham and R.O. Weller. "Neuropathology of human Alzheimer disease after immunization with amyloid-β peptide: a case report". Nat. Med. 9 (2003) 448-452
17: A.M. Cataldo, C.M. Peterhoff, S.D. Schmidt, N.B. Terio, K. Duff and M. Beard. "Presenilin mutations in familial Alzheimer disease and transgenic mice models accelerate neuronal lysosomal pathology".J. Neuropathol. Exp. Neurol. 63 (2004) 821-830.

## Claims

1. A method for the diagnosis and/or prognosis of AD comprising:
- determining the expression level of a gene encoding a lysosomal marker in a biological sample
- comparing said expression level with a reference value
in which the alteration of said level is indicative of AD.

2. A method for the diagnosis and/or prognosis of AD according to claim 1, wherein the gene encoding a lysosomal marker is Lamp-1.

3. A method for the diagnosis and/or prognosis of AD according to claim 1, wherein the gene encoding a lysosomal marker is Lamp-2.

4. A method for the diagnosis and/or prognosis of AD according to any of claims 1-3, wherein the biological sample is a tissue.

5. A method for the diagnosis and/or prognosis of AD according to any of claims 1-3, wherein the biological sample is a body fluid.

6. A method for the diagnosis and/or prognosis of AD according to claim 5, wherein the fluid comprises cerebrospinal fluid.

7. A method for the diagnosis and/or prognosis of AD according to any of claims 1-3, wherein the determination of the expression level of the gene encoding a lysosomal marker is carried out by means of measuring the amount of mRNA encoded by said gene or fragments thereof.

8. A method for the diagnosis and/or prognosis of AD according to claim 7, wherein the measurement of the amount of mRNA is carried out by means of RT-PCR amplification.

9. A method for the diagnosis and/or prognosis of AD according to claim 7, wherein the measurement of the amount of mRNA is carried out by means of DNA biochips.

10. A method for the diagnosis and/or prognosis of AD according to any of claims 1-3, wherein the determination of the expression level of the gene encoding a lysosomal marker is carried out by means of measuring the amount of protein encoded by said gene and/or fragments thereof.

11. A method for the diagnosis and/or prognosis of AD according to claim 10, wherein the measurement of the amount of protein is carried out by means of Western Blot.

12. A method for the diagnosis and/or prognosis of AD according to claim 10, wherein the measurement of the amount of protein is carried out by means of protein chips.

13. A method for the diagnosis and/or prognosis of AD according to claim 10, wherein the measurement of the amount of protein is carried out by means of immunohistochemistry.

14. A kit for the diagnosis and/or prognosis of AD comprising the necessary reagents for the determination of the expression level of the gene encoding a lysosomal marker according to any of claims 1 to 13.

15. A method for the diagnosis and/or prognosis of AD according to any of claims 1-3, wherein the determination of the expression level of the gene encoding a lysosomal marker is carried out by means of an indicating substance binding specifically to the mRNA or to the protein encoded by said gene.

16. A method for the diagnosis and/or prognosis of AD according to claim 15, wherein the expression level of the lysosomal marker gene is determined by means of images.

17. A kit for the diagnosis and/or prognosis of AD according to any of claims 15 to 16 comprising a composition comprising an reporter substance binding specifically to the RNA or to the protein encoded by said gene, wherein said reporter substance is labeled with a labeling which can be detected by means of a detection method, and a physiologically acceptable carrier fluid.

18. The use of at least one gene encoding a lysosomal marker selected from Lamp-1 and Lamp-2 as a genetic marker for the diagnosis of AD.
